# FASCICULE DE BREVET EUROPEEN

(11) **EP 3 655 771 B1**
(45) Date de publication et mention de la délivrance du brevet: **07.06.2023**
(21) Numéro de dépôt: 18739567.8
(22) Date de dépôt: 18.07.2018
(51) Int. Cl.: G01N 33/18

(54) **PROCEDE DE DETERMINATION DE LA PRESENCE D'UN CONTAMINANT DANS UN LIQUIDE**
VERFAHREN ZUR DETEKTION DER ANWESENHEIT EINER VERUNREINIGUNG IN EINER FLÜSSIGKEIT
METHOD FOR DETECTING THE PRESENCE OF A CONTAMINANT IN A LIQUID

(30) Priorité: 18.07.2017 FR 1756815
(43) Date de publication de la demande: 27.05.2020
(73) Titulaire: Institut national de recherche pour l'agriculture, l'alimentation et l'environnement, 75007 Paris (FR); View Point, 01390 Civrieux (FR)
(72) Inventeur: CHAUMOT, Arnaud, 69001 Lyon (FR); DAUPHIN, Maxime, 01430 Ceignes (FR); DECAMPS, Alexandre, 34610 Saint-Gervais-Sur-Mare (FR); GEFFARD, Olivier, 17180 Perigny (FR); MOULIN, Florian, 69004 Lyon (FR); NEUZERET, Didier, 01600 Sainte Euphemie (FR); QUEAU, Hervé, 69720 Saint-Bonnet de Mure (FR)
(74) Mandataire: Regimbeau
(86) Numéro de dépôt international: PCT/EP2018/069523
(87) Numéro de publication internationale: WO 2019/016273

(56) Documents cités:
- WO-A1-2008/150096
- CN-A- 101 059 493
- US-A- 4 164 199
- US-A- 4 722 371
- JEON ET AL: "Development of a new biomonitoring method to detect the abnormal activity of Daphnia magna using automated Grid Counter device", SCIENCE OF THE TOTAL ENVIRONMENT, ELSEVIER, AMSTERDAM, NL, vol. 389, no. 2-3, 24 octobre 2007 (2007-10-24), pages 545-556, XP022349616, ISSN: 0048-9697, DOI: 10.1016/J.SCITOTENV.2007.09.015

## Description

L'invention concerne un procédé autonome d'évaluation en ligne de la toxicité de solutions aqueuses par analyse du comportement locomoteur de macroinvertébrés aquatiques benthiques de petite taille (0.1cm-5cm) maintenus en état léthargique.

### CONTEXTE ET ARRIERE-PLAN TECHNOLOGIQUE

La qualité de la ressource en eau est une préoccupation sociétale majeure à la fois du point de vue de la préservation de l'environnement mais aussi de la santé. C'est pourquoi les états membres de l'Union Européenne se sont fixés des lignes directrices capitales à savoir une épuration des eaux de rejet (**91/271/CEE**), limiter l'entrée dans l'environnement de substances prioritaires (**2013/39/UE**), réduire à la source l'entrée de contaminants et atteindre un « bon état » des masses d'eau sur l'ensemble du territoire (**DCE 2000/60/CE**).

Bien que la majorité des déversements des rejets urbains et industriels passe par une phase de traitement, ces derniers sont aujourd'hui conçus pour éliminer principalement la matière organique et limiter l'eutrophisation des milieux aquatiques liée à l'apport d'azote et de phosphore selon la sensibilité de l'hydrosystème récepteur. Toutefois, les rejets restent un vecteur important d'entrée de contaminants (micropolluants) dans l'environnement (**Cairns et Van Der Schalie, 1980**), constituant un enjeu fort aujourd'hui pour les gestionnaires du traitement des eaux usées et constituent un élément clef dans la protection des milieux et la préservation de la ressource en eau.

Le principe de « surveillance des systèmes de collecte des eaux usées et des stations d'épuration en vue d'en maintenir et d'en vérifier l'efficacité » par les collectivités est institué depuis 1991 par la directive européenne sur le traitement des Eaux Résiduaires Urbaines (ERU). Les récentes évolutions de la législation française, notamment par l'arrêté ministériel du 21 juillet 2015 impose une approche, à l'échelle du système d'assainissement, d'autoévaluation. Aujourd'hui, ces systèmes d'autoévaluation reposent sur des prélèvements ponctuels, moyennés sur 24h, ne permettant pas de connaître le fonctionnement dynamique de la station.

Dans l'anticipation d'une législation plus sévère et dans le but de limiter l'apport de contaminants dans l'environnement, une réelle demande d'outil d'analyse de la qualité des rejets en ligne est formulée de la part des gestionnaires de l'eau public et privé. Au-delà de l'aspect réglementaire, le développement d'outils d'auto-surveillance constituerait une réelle opportunité pour affiner les connaissances des gestionnaires sur leur rejet, notamment leur évolution dans le temps, et de caractériser en temps-réel leur qualité pour optimiser la gestion des systèmes d'assainissement.

Pour obtenir une évaluation globale, robuste et significative du point de vue environnemental, il est choisi de s'appuyer sur des valeurs écotoxicologiques. Les analyses chimiques, avec les techniques actuelles et pour la caractérisation des rejets, semblent difficiles en raison des fortes hétérogénéités temporelles et du grand nombre de molécules qui les compose (Cairns et Van Der Van, 1980 ; Sornom, 2012). De plus, le simple fait de connaître les concentrations de quelques produits ne suffit pas pour produire des informations de gestion utile (Cairns et Van Der Schalie, 1980), c'est pourquoi l'utilisation d'outils biologiques, qui intègrent tous les facteurs du milieu et la part biodisponible des contaminants présents dans le rejet est plus prometteuse. Par ailleurs, évaluer en ligne la qualité des rejets sans passer par des prélèvements ponctuels comme c'est le cas aujourd'hui pour pratiquer des tests de toxicité normalisés Afnor permettrait d'éviter un grand nombre de limites actuelles de la bioévaluation (évaluation ponctuelle, dénaturation des échantillons lors du transport et de leur conservation, réponse longue à acquérir).

L'utilisation d'outils biologiques, notamment l'analyse du comportement locomoteur pour évaluer la qualité toxique des rejets est une solution robuste et adéquate puisque ce biomarqueur est reconnu pour être ubiquiste, généraliste et avoir un temps très court de réponse (**Blaxter et Hallers-Tjabbes, 1992 ; Hellou, 2011**). Ce biomarqueur est basé notamment sur des réponses de chémoréceptions de l'organisme (**Gerhardt, 1996**). Le suivi en ligne du comportement locomoteur permet de détecter de plus faibles concentrations de contaminants que les études de létalité conventionnelles (10-1000 fois plus sensibles) (**Hellou et al., 2008 ; Robinson 2009**) et de connaître, avec un pas de temps faible, la toxicité des rejets avant leurs déversements dans le milieu naturel.

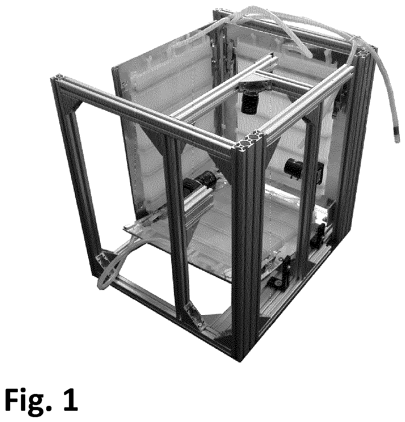

L'idée d'utiliser des macroorganismes aquatiques pour évaluer la toxicité en continu de milieux aquatiques n'est pas nouvelle. Des dispositifs ont commencé à se développer en se basant sur le suivi de poissons (**Henderson et Pickering, 1963**) puis se sont affinés pour suivre des invertébrés comme le gammare, l'hydropsyche (**Gerhardt, 1996**), la daphnie (**Ren et *al*. 2007 ; Jeon et *al*., 2008, Chevalier, 2014**). L'ensemble de ces travaux assoit la pertinence de l'utilisation du biomarqueur de l'activité locomotrice dans l'évaluation précoce et sensible de contaminant dans l'eau. A présent que la preuve de pertinence n'est plus à faire, il est nécessaire d'adapter ce concept d'outils à l'analyse des eaux de rejet et de les rendre autonomes. Pour ce faire, d'importants verrous doivent être levés à savoir la conception d'un dispositif permettant le suivi à long terme de l'ordre de plusieurs semaines, de plusieurs macroorganismes simultanément et adapté pour recevoir des eaux de rejets (Figure 1). Il est également nécessaire de développer un signal toxique adapté à l'évaluation en ligne et sur site des rejets urbains et industriels. Ce dernier doit détecter de faibles concentrations de contaminants tout en excluant des facteurs de confusion intrinsèques aux conditions de l'exposition, à savoir la présence de vibrations sur les sites industriels ou urbains (pompe, activité humaine, passage de véhicule, etc.) et les variations naturelles de compositions physico-chimiques en ions majeurs de l'eau de rejet, qui peuvent avoir une incidence sur le comportement locomoteur des bioessais.

Un des procédés d'analyse consiste à surveiller ces macroorganismes vivants pendant toute la durée de l'exposition pour en étudier le comportement, et en déduire en continu la toxicité du milieu testé tout au long de l'exposition. Pour ce faire, un organisme est disposé dans un récipient transparent au moins sur une face d'observation, et des images du récipient sont acquises à travers la face d'observation par un imageur disposé en face de la face d'observation.

Toutefois, s'agissant d'êtres vivants, il existe un certain aléa quant à la réaction de l'organisme vivant à l'exposition du rejet étudié et potentiellement toxique, voire même des pertes inévitables de ceux-ci. Il est donc nécessaire d'étudier plusieurs êtres vivants simultanément et de faire un traitement statistique des résultats pour gommer ces aléas. Il n'est cependant pas opportun généralement de disposer plusieurs êtres vivants dans le même récipient puisque les macroorganismes peuvent alors interférer entre eux, ce qui peut compliquer l'interprétation des résultats de la surveillance.

Le principe du procédé de l'invention repose sur la mise en état léthargique des macrorganismes avant leur exposition dans des conditions d'observation stables et contrôlées. Cette léthargie correspond ici à un comportement locomoteur minimal de l'espèce observée et une absence d'activité de nutrition (extrême réduction de la locomotion, absence de comportement de recherche alimentaire et de prise alimentaire). La présence de contaminants dans le milieu d'exposition se traduit alors par une sortie de cet état de léthargie du fait des capacités de chémoréception des organismes utilisés et du comportement d'évitement face au stress chimique qui s'en suit. Le procédé de l'invention ne nécessite a priori pas de liquide témoin. Pour se garantir de la robustesse de la détection de cette réponse de sortie de l'état léthargique, il est avantageux d'étudier un lot d'individus homogène au regard de leurs caractéristiques biologiques : individus calibrés en sexe, taille, statut reproducteur, provenant d'une population unique, de préférence stabulés au laboratoire avant la transplantation dans les chambres d'observation. Il est également nécessaire que les êtres vivants conservent cette homogénéité au cours de la période d'observation (absence de mue, de ponte, de croissance). Cet état léthargique ne peut pas être atteint par des organismes comme la daphnie qui sont par définition toujours en mouvement et une absence de locomotion est synonyme de mort. Les organismes pouvant atteindre cet état sont des macroinvertébrés aquatiques benthiques.

Le procédé de l'invention est particulièrement avantageux car il permet d'évaluer en ligne la toxicité de façon autonome entre 2 et 30 jours de suivi avec une fréquence d'évaluation sur des pas de temps faibles (entre 2-120 minutes).

### FIGURES

Figure 1 : Photographie du toximètre développé par Irstea-ViewPoint
Figure 2 : Graphique d'enregistrement de l'évolution de l'indice locomoteur lors de changements brutaux de conductivité
Figure 3 : Evolution de l'indice locomoteur sur 3 semaines lors d'ajout ponctuel de solution contaminée au Méthomyl (100µg/L)
Figure 4 : Suivi de la qualité toxique d'eau traitée à la sortie d'une station d'épuration durant 28 jours
Figure 5 : photographie de *Gammarus fossarum* (Amphipode de la famille des Gammaridae 8-30mm)
Figure 6 : photographie de *Erpobdella testacea* (Annelide sous classe des Hirudinea (sangsue) de 15-40mm)
Figure 7 : photographie de *Radix auricularia* (Gastéropode pulmoné de 6-30mm)
Figure 8 : Photographie d'une ZebraBox

### DEFINITIONS

Au sens de la présente invention, « mouvement » signifie changement de position dans l'espace. Ce changement peut se caractériser de multiples manières comme la mesure d'une distance parcourue, une vitesse, une rotation, les changements d'orientation d'un corps entier ou d'une de ses parties (tête, patte, etc.).

Au sens de la présente invention, « comportement » signifie mesure de mouvement par unité de temps.

Au sens de la présente invention, état de léthargie signifie l'état d'un lot d'individus caractérisé par :
- un comportement moyen (i) constant sur une durée de six heures a minima, de préférence 12 heures, et (ii) diminué a minima de 60%, de préférence de 80% et de façon avantageuse de 90% par rapport au comportement mesuré pendant la première heure d'observation du lot d'individus,
- une absence d'activité de nutrition.

Au sens de la présente invention, sortie de la léthargie signifie l'augmentation du comportement moyen d'un lot d'individus d'au moins un facteur deux sur une période d'au moins 10 minutes.

Au sens de la présente invention, « stabulation » signifie maintien en conditions contrôlées (pH, alimentation, conductivité, luminosité) des macroinvertébrés aquatiques benthiques, afin d'homogénéiser le lot d'individus utilisés lors de l'expérimentation (réserves énergétiques, état physiologique) et éliminer les individus affaiblis lors du prélèvement, et les conditionner aux conditions d'exposition futures.

Au sens de la présente invention, « indice locomoteur » correspond à une distance moyenne calculée selon les contextes d'étude (terrain ou laboratoire) sur les 10 à 80% des valeurs les plus faibles de distances individuelles parcourues. L'indice locomoteur est la moyenne de ces distances les plus faibles. Cet indice locomoteur est calculé sur un pas de temps d'intégration pouvant être choisi entre 2 et 120 minutes. Les valeurs de distances parcourues sont mises au carré avant le calcul de l'indice, ainsi l'indice locomoteur s'exprime en mm²/unité de temps. Au sens de la présente invention, « contaminant » signifie l'ensemble des substances chimiques (produits phytosanitaires, métaux lourds, HAPs, polluants organiques, résidus médicamenteux, etc.) capable d'engendrer un comportement d'évitement chez les macroinvertébrés aquatiques benthiques exposés.

### DESCRIPTION DETAILLEE

L'invention concerne un procédé de détermination de la présence d'un contaminant dans l'eau, comprenant les étapes suivantes :
a) un lot de macroinvertébrés aquatiques benthiques de petite taille sont conditionnés afin qu'ils entrent dans un état léthargique
b) lesdits macroinvertébrés aquatiques benthiques sont immergés dans l'eau, chaque organisme étant isolé individuellement,
c) le comportement de chaque organisme aquatique est enregistré en continu lors de l'exposition ;
d) les données de comportement sont analysées au moyen d'un dispositif de traitement pour déterminer le comportement des macroinvertébrés aquatiques benthiques
e) la présence de contaminants toxiques est déterminée en fonction du comportement des macroinvertébrés aquatiques benthiques ;
caractérisé en ce que l'analyse des données comprend la détermination, pour chacune des espèces desdits macroinvertébrés aquatiques benthiques, d'un comportement moyen représentatif des déplacements des macroinvertébrés aquatiques benthiques de cette espèce pendant une durée déterminée, les macroinvertébrés aquatiques benthiques étant placés pendant la période d'observation dans des conditions dans lesquelles l'absence de toxicité se traduit par le maintien de l'ensemble du lot de macroinvertébrés aquatiques benthiques dans un état léthargique, lesdites conditions étant favorables au maintien en vie desdits macroinvertébrés aquatiques benthiques et comprenant une absence d'apport de nourriture pendant la période d'observation,
et en ce que la détermination de la présence de toxicité comprend la comparaison dudit comportement moyen par rapport au comportement moyen de l'état léthargique, la présence de toxicité étant avérée lorsque le comportement moyen augmente d'au moins un facteur deux sur une période d'au moins 10 minutes.

Les macroinvertébrés aquatiques benthiques peuvent être placés dans des conditions adéquates d'absence de stimuli permettant d'atteindre l'état léthargique. Les espèces mises en oeuvre dans le procédé de l'invention doivent pouvoir être maintenues en vie sur une période d'au moins une semaine dans un milieu cloisonné, en conditions lumineuses et température constantes, sans apport de nourriture, sans changement de stade de développement (mue, métamorphose, ...) ou événements de reproduction (ponte, reproduction asexuée).

Le procédé de l'invention est particulièrement avantageux car autonome sur une durée prolongée d'au moins deux jours, de préférence d'au moins deux semaines et de manière particulièrement préférée de 30 jours.

Chaque macroinvertébré aquatique benthique est isolé individuellement dans une chambre d'observation afin de s'assurer que les données d'un individu ne sont pas perturbées par la présence d'un ou plusieurs autres individus et d'assurer une évaluation robuste en ayant un grand nombre de réplicats.

Avantageusement, au moins 12 macroinvertébrés aquatiques benthiques, de préférence au moins 16 macroinvertébrés aquatiques benthiques, par espèce sont inclus dans le procédé de l'invention et observés (Figure 1).

De préférence, à l'étape c) le comportement de chaque macroinvertébré aquatique benthique est enregistré par acquisition simultanée d'images au moyen d'un imageur ou par mesure de l'impédance (« Monitoring Behavioural Responses to Metals in Gammarus pufex (L.) (Crustacea) with Impeadance Conversion, Almut Gerhardt, Environ. Sci. And Pollut. Res. 2(1), 1995).

Les images sont analysées et le calcul de l'indice locomoteur est calculé en continu et transmis en ligne (sur un site internet) en temps-réel.

De préférence, le procédé selon l'invention est précédé d'une étape préalable à l'étape a) de sélection des macroinvertébrés aquatiques benthiques pour que chaque espèce représente une population d'êtres vivants la plus homogène possible : calibrés en sexe, taille, statut reproducteur, non-parasités. Avantageusement, les individus de chaque espèce proviennent d'une population unique.

Il est également nécessaire que cette population d'êtres vivants conserve son homogénéité : pas de mue, pas de ponte, pas d'alimentation.

Les réserves énergétiques des macroinvertébrés aquatiques benthiques choisis permettent leur maintien en vie pendant toute la durée de mise en oeuvre du procédé selon l'invention sans apport de nourriture, de préférence durant au moins une semaine, de manière particulièrement préférée durant un mois. Ainsi, l'acquisition des images n'est pas perturbée par un apport de nourriture dans le liquide. Les daphnies ne sont pas adaptées au procédé de l'invention puisque ne supportant pas une diète prolongée.

De préférence, les macroinvertébrés aquatiques benthiques sont préalablement sélectionnés pour qu'ils représentent une population homogène en taille, en sexe et non-parasitée.

On privilégiera des individus qui n'auront pas de petits pendant la durée du procédé, par exemple de sexe non-femelle ou immatures sexuellement afin d'éviter la production de petits dans le liquide qui perturberont l'acquisition des images.

On privilégiera des individus qui ne muent pas pendant la durée du procédé.

De préférence encore, les macroinvertébrés aquatiques benthiques seront préalablement à l'étape b) stabulés afin de les acclimater aux conditions de la mesure.

Lesdits macroinvertébrés aquatiques benthiques peuvent appartenir à la même espèce ou être choisis parmi plusieurs espèces, de préférence d'ordres phylogénétiques différents, de préférence jusqu'à trois espèces.

Lesdits macroinvertébrés aquatiques benthiques sont de petite taille, inférieure à 5 cm, de préférence inférieure à 2 cm, de manière particulièrement préférée inférieure à 1 cm.

Avantageusement, on privilégiera des gastéropodes, des sangsues et/ou des crustacés.

De préférence, l'espèce de gastéropode est du genre Radix, de préférence *Radix auricularia.*

De préférence, l'espèce de sangsue est de l'ordre des Arhynchobdellida, de préférence de la famille des Erpobdellidae, par exemple *Erpobdella testacea.*

De préférence, l'espèce de crustacé est un amphipode, de préférence de la famille des Gammaridae, par exemple *Gammarus fossarum.*

Avantageusement, lesdits macroinvertébrés aquatiques benthiques présentent un taux de survie de plus de 80% à 30 jours de diète dans les conditions physico-chimiques du procédé de l'invention sans contaminant.

De préférence, les images sont acquises à l'étape c) toutes les 66 mS (15 Hertz) et intégrées sur 20 secondes.

### EXEMPLES

Dans les exemples, sont exposés uniquement des macroinvertébrés aquatiques benthiques calibrés en termes de taille, sexe, non parasités, provenant d'une population unique et stabulés deux semaines en laboratoire. Les macroinvertébrés aquatiques benthiques sont placés dans un encagement sous caméra où seize individus sont placés seuls dans un volume de 50ml d'eau. L'encagement est construit à partir de matériaux neutres (polypropylène) et sans la moindre utilisation de produits toxiques (pas de colle). Ces encagements (Figure 1) sont branchés à une alimentation de liquide préalablement bullée, thermo-régulée et filtrée puis circule dans la chambre qui reçoit l'effluent à un débit de 6L/h.

Pendant la durée d'exposition (30 jours), les macroinvertébrés aquatiques benthiques sont exposés dans des conditions favorables à leur maintien (13±2°C, saturée d'oxygène et sans prédateur) et mis en diète pour homogénéiser le comportement (les réserves énergétiques des macroinvertébrés aquatiques benthiques choisis permettent dans ces conditions leur maintien en vie durant un mois).

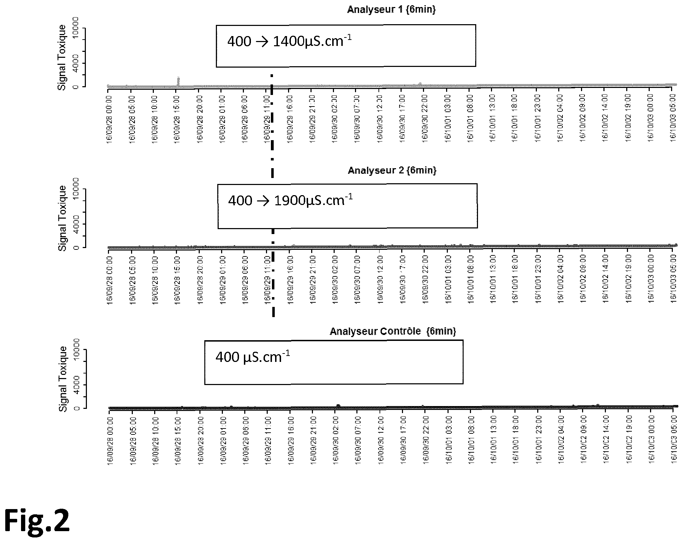

L'une des applications possibles de l'évaluation en ligne de la toxicité d'un liquide par l'analyse du comportement de fuite de macroinvertébrés aquatiques benthiques est l'analyse des eaux de rejets urbains ou industriels en sortie de traitement. L'outil est conçu pour être transportable et effectuer les mesures sur site afin d'avoir une évaluation en direct et en continu. Une première expérience a été effectuée dans une station d'épuration afin d'intégrer dans le suivi de la toxicité des eaux traitées tous les facteurs qui peuvent avoir une influence sur le comportement des macroinvertébrés aquatiques benthiques en les activant et biaiser l'évaluation de la toxicité (vibrations issues des pompes, des activités humaines, passages de véhicules, etc.). De plus, les eaux traitées présentent des variations naturelles physico-chimiques dans le temps (selon les déversements dans le bassin versant, épisodes pluvieux, fonctionnement station d'épuration, etc.). Pour mesurer l'influence de ces deux facteurs, à savoir les perturbations sur site et les variations physico-chimiques, trois toximètres ont été placées dans une station d'épuration connectées avec de l'eau non-contaminée durant 9 jours. Les deux premiers jours permettent aux sondes biologiques d'atteindre un état léthargique caractérisé par une inactivité qui ne sont pas présentés dans ce résultat. A la suite de ces deux jours, le suivi de la toxicité démarre. Au quatrième jour, il a été ajouté de manière brutale dans les toximètres 1 et 2 des solutions enrichies en sels afin de passer à une conductivité de 400 µs.cm⁻¹ à respectivement 1400 et 1900µS.cm⁻¹. Ces changements de conductivité ont été mesurés en continu par un conductimètre. Les suivis des trois toximètres sont présentés dans la Figure 2. L'indice locomoteur de chacun des toximètres ne présente aucune modification durant les fortes variations de conductivités où durant l'ensemble de l'expérience due aux perturbations de vibrations engendrées par l'activité intrinsèque de la station. L'expérience montre que la méthode d'analyse de la toxicité développée exclut deux importants facteurs de confusion à savoir les variations dans le temps de la conductivité et la présence de perturbations dues à l'activité de la station d'épuration. Cet outil et la méthode de calcul sont ainsi appropriés pour l'évaluation en ligne de la toxicité des eaux traitées.

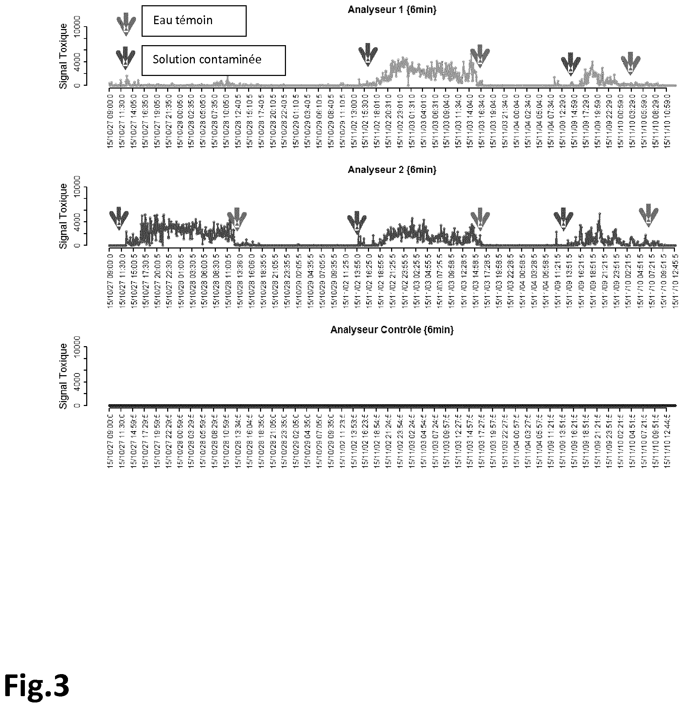

Une expérimentation a été menée pour vérifier la détection de toxicité par le toximètre et sa méthode de calcul de l'indice locomoteur. Trois toximètres sont connectés à une eau non-contaminée puis le toximètre 1 reçoit 1 épisode de contamination de 20-24h aux semaines 1, 2 et 3, le toximètre 2 reçoit un épisode de contamination de 20-24h aux semaines 2 et 3 et le toximètre 3 sert de témoin afin de s'assurer que les potentielles activations des sondes biologiques ne soient pas issues d'une perturbation (forte vibration). L'épisode de contamination correspond à l'ajout d'un insecticide, le méthomyl, à une concentration de 100µg.L-1. Cette concentration est choisie élevée pour être sûr d'observer des effets sur le comportement locomoteur des macroinvertébrés aquatiques benthiques sélectionnés (LC50 chez la Daphnie à 28µg.L⁻¹ après 48h d'exposition). Les trois suivis sont présentés dans la Figure 3. Il est observé que la méthode de suivi permet de détecter une toxicité du produit en 3h, et cela même après plusieurs épisodes de contamination. Il est important de souligner que ces concentrations, dans un environnement d'eau de sortie de station d'épuration peuvent être habituelles.

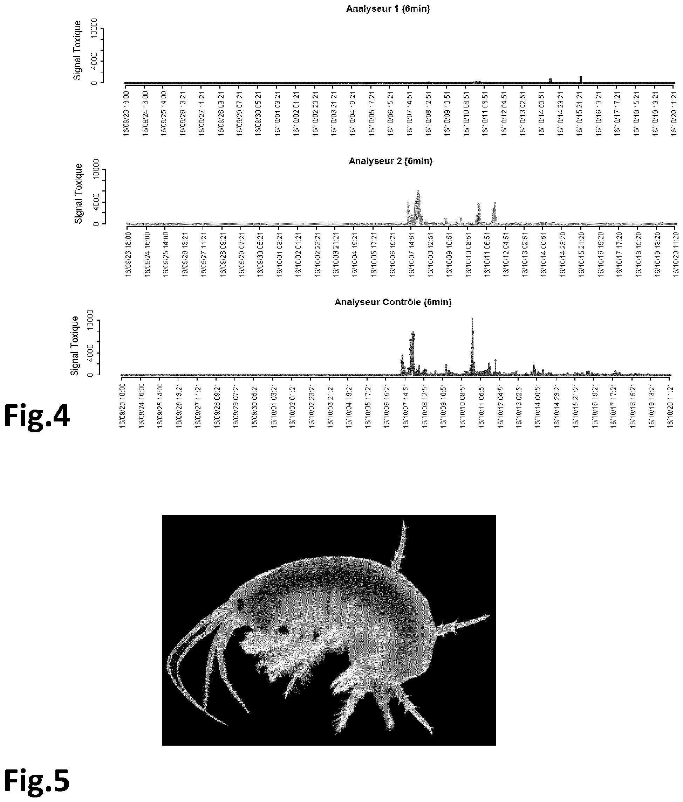

Après avoir ainsi validé que l'indice locomoteur développé détecte la toxicité de contaminants à des concentrations environnementales et qu'il est insensible aux variations de teneurs en ions majeurs, un suivi de la toxicité d'une eau en sortie de traitement de station d'épuration a été effectué. Deux toximètres ont été connectés en ligne sur les rejets d'une station d'épuration et un dernier a été connecté à une eau non-contaminée afin d'assurer une condition témoin sur sites. Les suivis sont présentés dans la Figure 4. Ce suivi montre que le dispositif développé permet de suivre en totale autonomie durant un mois l'activité locomotrice des macroinvertébrés aquatiques benthiques dans le toximètre alors qu'il est connecté à une eau en sortie de traitement (eau trouble, forte teneur en MES). Le témoin présente un indice locomoteur constant et très faible le long du suivi correspondant au maintien de l'état léthargique du lot d'individus exposés sur site. En contraste, les deux toximètres, connectés à la même eau de rejet en parallèle de ce témoin, détecte simultanément deux épisodes de sortie de l'état léthargique traçant une dégradation de la qualité toxique des effluents ; un premier le 7 octobre 2016 de 11h30 à 16h et un second le 10 octobre 2016 de 10h à 12h30. L'outil et la méthode développés permettent ainsi de suivre en ligne la qualité toxique d'une solution aqueuse en autonomie par l'analyse du comportement de fuite de macroinvertébrés aquatiques benthiques.

Les étapes de cette évaluation sont successivement la sélection des espèces, le prélèvement, la stabulation pendant une quinzaine de jours, le tri (calibration), la mise en chambre d'encagement, mise en eau du toximètre à l'effluent et l'analyse individuelle en continu du comportement locomoteur des macroinvertébrés aquatiques benthiques.

### EXEMPLE 1 : SELECTION DES ESPECES, PRELEVEMENT, STABULATION ET TRI DES MACROINVERTÉBRÉS AQUATIQUES BENTHIQUES

### I. MATERIEL

- **SELECTION DES ESPECES :** troubleau, colonne de tamis, seaux en polyéthylène, installation pour stabulation à 13°C en aquarium de 16L.
- **PRELEVEMENT :** troubleau, colonne de tamis de vide de maille 1.6mm, 2 mm, 2,5 mm et un dégrilleur, seaux en polyéthylène.
- **STABULATION** : installation pour stabulation à 13 °C en aquariums de 16L.
- **TRI :** table lumineuse ; plats transparents en pyrex ; maille ; épuisette ; seaux en polypropylène (type seaux alimentaires), plaque en polystyrène.

### II. METHODE

### 11.1. SELECTION DES ESPECES

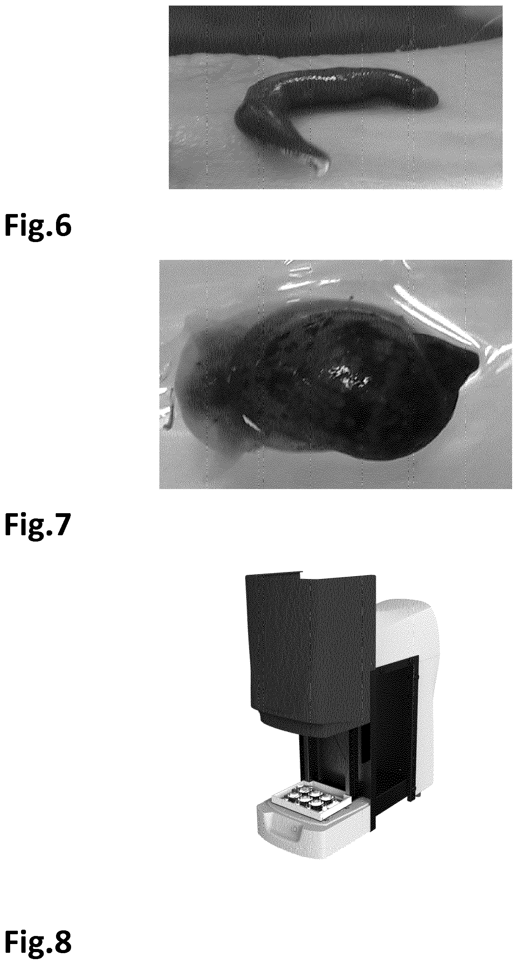

La sélection des espèces est une étape primordiale puisqu'elle influe directement sur la pertinence de l'évaluation toxique. Les critères de sélection vérifiés sur le cheptel d'espèces testé au laboratoire sont décrits ci-dessous avec leur justification :
- correspondance entre la zone de répartition de l'espèce et le site d'étude pour ne pas apporter de pollution biologique dans les milieux aquatiques en cas d'accident.
- présent en forte quantité dans l'environnement toute l'année afin de ne pas être limité à un moment donné par la réserve d'organisme.
- pouvant survivre plus de 30 jours en diète car le toximètre se veut indépendant sur un mois et que la présence d'une réserve de nourriture altérerait la qualité du vidéo-tracking.
- ordre phylogénétique différent, représentatif de la biodiversité rivulaire, et sensible à la présence de contaminants. En augmentant la diversité des espèces, le spectre de sensibilité aux contaminants est plus important avec des sensibilités différentes. L'évaluation à partir de ce toximètre se révèle pertinent puisqu'il possède une représentativité environnementale.
- comportement mesurable en continu et dans le temps afin d'obtenir un diagnostic en direct et prévenir rapidement les gestionnaires en cas de problèmes.
- une espèce placée à horizontale et deux autres à la verticale pour optimiser la place dans le toximètre (cube de 30cm).
- facilement manipulable, transportable et stabulable afin que la mise en place demeure facile. Les macroinvertébrés aquatiques benthiques sélectionnés pour l'évaluation toxique des rejets en Europe sont *Gammarus fossarum* (Figure 5), qui est placé dans l'encagement horizontal, *Erpobdella testacea* (Figure 6) et *Radix auricularia* (Figure 7) sont placés dans les encagements verticaux.

### 11.2. PRELEVEMENT DES MACROINVERTÉBRÉS AQUATIQUES BENTHIQUES

Les macroinvertébrés aquatiques benthiques proviennent d'une population source présentant de fortes densités tout au long de l'année. Dans l'exemple développé, les organismes sont issus de l'ancienne cressonnière du Bugey sur la commune de Saint-Maurice-de-Remens, dans le département de l'Ain qui a été définie comme station de prélèvement.

Le prélèvement est effectué à l'aide d'un troubleau en régime stabilisé dans les différents habitats de chacune des espèces.

L'échantillon recueilli au fond du troubleau est ensuite déposé sur la colonne de tamis de vide de maille de 1,60mm, 2,00 mm, 2,50 mm et du dégrilleur. Un rinçage sur la colonne de tamis est ensuite effectué à l'aide d'un seau, puis directement dans la rivière. Les macroinvertébrés aquatiques benthiques sont contenus dans les différents tamis.

Cette opération est répétée plusieurs fois selon la quantité de macroinvertébrés aquatiques benthiques souhaitée.

### 11.3. STABULATION DES MACROINVERTÉBRÉS AQUATIQUES BENTHIQUES AVANT IMPLANTATION DANS LE TOXIMETRE

Avant stabulation, chaque espèce est séparée des autres macroinvertébrés aquatiques benthiques, des débris végétaux et minéraux. Ensuite, ils sont placés dans des aquariums de 16 L contenant de l'eau du site renouvelée au goutte à goutte (volume renouvelé quatre fois par jour) par une eau douce de forage d'une conductivité de 450 µS.cm-1, oxygénée par l'intermédiaire d'un bullage continu. L'aquarium est placé dans un bain marie thermo-régulé à 13 ± 1 °C, exposé à une photopériode de 14 heures de jour / 10 heures de nuit. L'alimentation est effectuée *ad libitum* et la durée de stabulation préconisée est de deux semaines. La stabulation a pour objectif d'éliminer les macroinvertébrés aquatiques benthiques affaiblis lors du prélèvement, d'assurer une certaine « homogénéité physiologique » des macroinvertébrés aquatiques benthiques utilisés quelle que soit la saison (réserves énergétiques, taux d'activité comportementale comme la locomotion, l'alimentation), et de les acclimater aux futures conditions d'exposition. Les caractéristiques de l'eau de stabulation sont les suivantes : température = 13 ± 1 °C ; conductivité = 450 ± 50 µS.cm-1 ; pH = 7,6 ± 0,2 ; photopériode = 14h jour /10h nuit. Les macroinvertébrés aquatiques benthiques sont placés à l'obscurité les deux derniers jours avant l'installation dans le toximètre.

### II.4. TRI DES MACROINVERTÉBRÉS AQUATIQUES BENTHIQUES (CALIBRATION)

La préparation des macroinvertébrés aquatiques benthiques s'effectue au laboratoire quelques jours avant l'exposition. Les macroinvertébrés aquatiques benthiques sélectionnés sont des mâles (pour les gammares, les radix et sangsues sont hermaphrodites), non-parasités et de taille uniforme pour chaque espèce. Le tri s'effectue sur table lumineuse avec de l'eau du milieu de stabulation. Le sexage s'effectue à l'œil nu. Les macroinvertébrés aquatiques benthiques sont séparés et placés avec de la nourriture adaptée dans un encagement de polypropylène, matériaux sans effet toxique.

A l'aide d'une maille, les macroinvertébrés aquatiques benthiques sont triés selon les critères de taille, sexe et non parasité de chaque espèce puis les transférer dans le second plat. Seuls les gammares ayant juste mués, peuvent intégrer le toximètre afin d'éviter une activation due à une mue.

### ETAPE 2 : MISE EN PLACE DE L'EXPERIMENTATION

Une installation doit être montée sur le site d'étude afin de suivre le comportement locomoteur des macroinvertébrés aquatiques benthiques. Les macroinvertébrés aquatiques benthiques sont placés individuellement dans des cellules afin de suivre le comportement locomoteur des macroinvertébrés aquatiques benthiques. Le Toximètre ou les ZebraBox^{®} (Figure 8) (Viewpoint, http://www.viewpoint.fr/en/p/equipment/zebrabox) sont appropriés pour ce genre de suivi. La solution à tester est préalablement filtrée (<500µm), bullée et thermorégulée.

Les toximètres ou les ZebraBox^{®} sont connectés à la solution à tester et l'évaluation en ligne de la qualité toxique démarre 48h après la mise en place des sondes biologiques et de l'alimentation des outils de mesures (toximètre ou ZebraBox).

Chaque ZebraBox^{®} est équipée d'une caméra et le Toximètre est équipée de 3 caméras (une pour chaque face).

Les caméras filment en continu les déplacements individuels des macroinvertébrés aquatiques benthiques.

Un ordinateur traite les images et calcul en temps-réel l'indice locomoteur.

Une évaluation de la qualité toxique peut être fournie entre toutes les 2 et 120 minutes.

L'évaluation toxique en ligne se fait de façon autonome et peut durer entre 2 et 30 jours.

L'ordinateur connecté à internet communique en direct les résultats sur un site internet.

En cas de détection de toxicité, le responsable du fonctionnement de l'outil peut être averti

### (sms, mail, etc.)

Dans le cas d'une non-connexion, les données sont archivées dans une carte SD.

## Revendications

1. Procédé de détermination de la présence d'un contaminant dans l'eau, comprenant les étapes suivantes :
a) un lot de macroinvertébrés aquatiques benthiques d'une taille inférieure à 5 cm sont conditionnés afin qu'ils entrent dans un état léthargique
b) lesdits macroinvertébrés aquatiques benthiques sont immergés dans l'eau, chaque organisme étant isolé individuellement,
c) le comportement de chaque organisme aquatique est enregistré en continu lors de l'exposition ;
d) les données de comportement sont analysées au moyen d'un dispositif de traitement pour déterminer le comportement des macroinvertébrés aquatiques benthiques
e) la présence de contaminants toxiques est déterminée en fonction du comportement des macroinvertébrés aquatiques benthiques ;
où l'analyse des données comprend la détermination, pour chacune des espèces desdits macroinvertébrés aquatiques benthiques, d'un comportement moyen représentatif des déplacements des macroinvertébrés aquatiques benthiques de cette espèce pendant une durée déterminée, les macroinvertébrés aquatiques benthiques étant placés pendant la période d'observation dans des conditions dans lesquelles l'absence de toxicité se traduit par le maintien de l'ensemble du lot de macroinvertébrés aquatiques benthiques dans un état léthargique, lesdites conditions étant favorables au maintien en vie desdits macroinvertébrés aquatiques benthiques et comprenant une absence d'apport de nourriture pendant la période d'observation, ledit état léthargique étant **caractérisé par** :
- un comportement moyen (i) constant sur une durée de six heures a minima, de préférence 12 heures, et (ii) diminué a minima de 60%, de préférence de 80% et de façon avantageuse de 90% par rapport au comportement mesuré pendant la première heure d'observation du lot d'individus,
- une absence d'activité de nutrition.
et en ce que la détermination de la présence de toxicité comprend la comparaison dudit comportement moyen par rapport au comportement moyen de l'état léthargique, la présence de toxicité étant avérée lorsque le comportement moyen augmente d'au moins un facteur deux sur une période d'au moins 10 minutes.

2. Procédé selon la revendication 1, **caractérisé en ce que** à l'étape c) le comportement de chaque organisme aquatique est enregistré par acquisition simultanée d'images au moyen d'un imageur.

3. Procédé selon la revendication 1 ou 2, lesdits macroinvertébrés aquatiques benthiques étant choisis parmi une ou plusieurs espèces d'ordres phylogénétiques différents, de préférence de une à trois espèces.

4. Procédé selon l'une quelconque des revendications précédentes, lesdits macroinvertébrés aquatiques benthiques étant choisis parmi les gastéropodes, les sangsues et/ou les crustacés.

5. Procédé selon la revendication 4, lesdits gastéropodes étant du genre Radix, de préférence Radix auricularia.

6. Procédé selon la revendication 4 ou 5, lesdites sangsues étant de l'ordre des Arhynchobdellida, de préférence de la famille des Erpobdellidae, par exemple Erpobdella testacea.

7. Procédé selon l'une quelconque des revendications 4 à 6, lesdits crustacés étant des amphipodes, de préférence de la famille des Gammaridae, par exemple Gammarus fossarum,

8. Procédé selon l'une quelconque des revendications 1 à 7, comprenant une étape préalable à l'étape a) de sélection des macroinvertébrés aquatiques benthiques pour que chaque espèce représente une population homogène en taille, en sexe et non-parasitée.

9. Procédé selon l'une quelconque des revendications 1 à 8, chaque espèce de macroinvertébrés aquatiques benthiques provenant d'une population unique.

10. Procédé selon l'une quelconque des revendications 1 à 9, présentant une étape de stabulation préalable à l'étape b) desdits macroinvertébrés aquatiques benthiques afin de les acclimater aux conditions de la mesure.

11. Procédé selon l'une quelconque des revendications 1 à 10, lesdits macroinvertébrés aquatiques benthiques n'étant pas des macroinvertébrés aquatiques benthiques femelles.

12. Procédé selon l'une quelconque des revendications 1 à 11, lesdits macroinvertébrés aquatiques benthiques présentant un taux de survie de plus de 80% à 30 jours de diète.

## Patentansprüche

1. Verfahren zur Bestimmung einer Verunreinigung im Wasser, das folgenden Schritte umfasst:
a) eine bestimmte Menge von benthischen, aquatischen Makroinvertebraten, die kleiner als 5 cm sind, sind derart konditioniert, dass sie einen lethargischen Zustand erlangen
b) die benthischen, aquatischen Makroinvertebraten werden im Wasser versenkt, wobei jeder Organismus einzeln isoliert wird,
c) das Verhalten jedes aquatischen Organismus wird während der Exposition kontinuierlich aufgezeichnet;
d) die Verhaltensdaten werden mit einem Verarbeitungsgerät analysiert, um das Verhalten der benthischen, aquatischen Makroinvertebraten zu ermitteln
e) das Vorhandensein von toxischen Verunreinigungen wird anhand des Verhaltens der benthischen, aquatischen Makroinvertebraten ermittelt;
bei dem die Datenanalyse die Ermittlung eines für jede der Arten der benthischen, aquatischen Makroinvertebraten repräsentativen durchschnittlichen Verhaltens bei den Bewegungen der benthischen, aquatischen Makroinvertebraten dieser Art während eines bestimmten Zeitraums umfasst, wobei die benthischen, aquatischen Makroinvertebraten während des Beobachtungszeitraums unter Bedingungen gesetzt werden, unter denen sich das Fehlen einer Toxizität darin äußert, dass die Gesamtheit der benthischen, aquatischen Makroinvertebraten in einem lethargischen Zustand verbleibt. Diese Bedingungen sind günstig für den Lebenserhalt der benthischen, aquatischen Makroinvertebraten und umfassen eine fehlende Nahrungsaufnahme während des Beobachtungszeitraums. Dieser lethargische Zustand ist **gekennzeichnet durch**:
- ein durchschnittliches Verhalten, das (i) über einen Zeitraum von mindestens sechs Stunden, vorzugsweise 12 Stunden, konstant bleibt, und (ii) um mindestens 60 %, vorzugsweise um 80 % und vorteilhafterweise um 90 % gegenüber dem während der ersten Beobachtungsstunde gemessenen Verhalten der Individuenmenge verringert ist,
- eine fehlende Nahrungsaufnahme.
und bei dem die Ermittlung des Vorhandenseins von Toxizität den Vergleich des durchschnittlichen Verhaltens mit dem durchschnittlichen Verhalten im lethargischen Zustand umfasst, wobei das Vorhandensein von Toxizität nachgewiesen wird, wenn das durchschnittliche Verhalten über einen Zeitraum von mindestens 10 Minuten zumindest um den Faktor zwei zunimmt.

2. Verfahren nach Anspruch 1, das **dadurch gekennzeichnet ist, dass** in Schritt c) das Verhalten jedes aquatischen Organismus durch zeitgleiche Bilderfassung mit Hilfe eines Bildaufnehmers aufgezeichnet wird.

3. Verfahren nach Anspruch 1 oder 2, wobei die benthischen, aquatischen Makroinvertebraten aus einer oder mehreren Arten unterschiedlicher phylogenetischer Ordnung, vorzugsweise aus einer bis drei Arten, ausgewählt werden.

4. Verfahren nach einem der vorangegangenen Ansprüche, wobei die benthischen, aquatischen Makroinvertebraten aus Gastropoden, Blutegeln und/oder Krustentieren ausgewählt werden.

5. Verfahren nach Anspruch 4, wobei die genannten Gastropoden aus der Gattung Radix, vorzugsweise aus der Gattung *Radix auricularia,* stammen.

6. Verfahren nach Anspruch 4 oder 5, wobei die genannten Blutegel der Gattung der Arhynchobdellida, vorzugsweise der Familie der Erpobdellidae, z. B. der *Erpobdella testacea,* angehören.

7. Verfahren nach einem der Ansprüche 4 bis 6, wobei die Krustentiere Amphipoden sind, vorzugsweise aus der Familie der Gammaridae, z. B. *Gammarus fossarum.*

8. Verfahren nach einem der Ansprüche 1 bis 7, das vor dem Schritt a) zur Auswahl der benthischen, aquatischen Makroinvertebraten einen weiteren Schritt beinhaltet, damit jede Art eine homogene Population in Bezug auf Größe, Geschlecht und nicht parasitäre Eigenschaft bildet.

9. Verfahren nach einem der Ansprüche 1 bis 8, wobei jede Art der benthischen, aquatischen Makroinvertebraten aus einer einzigen Population stammt.

10. Verfahren nach einem der Ansprüche 1 bis 9, das eine Phase der Unterbringung der genannten benthischen, aquatischen Makroinvertebraten vor Schritt b) umfasst, um sie an die Bedingungen der Messung zu akklimatisieren.

11. Verfahren nach einem der Ansprüche 1 bis 10, wobei die benthischen, aquatischen Makroinvertebraten keine weiblichen, benthischen, aquatischen Makroinvertebraten sind.

12. Verfahren nach einem der Ansprüche 1 bis 11, wobei die benthischen, aquatischen Makroinvertebraten eine Überlebensrate von mehr als 80 % bei 30 Tagen Diät aufweisen.

## Claims

1. A method for determining the presence of a contaminant in water, comprising the following steps of:
a) conditioning a batch of benthic aquatic macroinvertebrates of less than 5 cm in size so that they enter a lethargic state
b) immersing said benthic aquatic macroinvertebrates in water, each organism being individually isolated,
c) continuously recording the behaviour of each aquatic organism during the exposure;
d) analysing the behavioural data by means of a processing device to determine the behaviour of the benthic aquatic macroinvertebrates
(e) determining the presence of toxic contaminants as a function of the behaviour of the benthic aquatic macroinvertebrates;
wherein analysing the data includes determining, for each of the species of said benthic aquatic macroinvertebrates, an average behaviour representative of movements of the benthic aquatic macroinvertebrates of that species for a determined duration, the benthic aquatic macroinvertebrates being placed during the observation period under conditions in which the absence of toxicity results in the maintenance of the entire batch of benthic aquatic macroinvertebrates in a lethargic state, said conditions being favourable to keep said benthic aquatic macroinvertebrates alive and comprising a lack of food intake for the observation period, said lethargic state being **characterised by**:
- an average behaviour (i) constant for a duration of six hours as a minimum, preferably 12 hours, and (ii) reduced by 60% as a minimum, preferably 80% and advantageously 90% in relation to the behaviour measured during the first hour of observation of the batch of individuals,
- a lack of nutrition activity.
and in that determining the presence of toxicity comprises comparing said average behaviour with the average behaviour of the lethargic state, the presence of toxicity being proven when the average behaviour increases by at least a factor of two over a period of at least 10 minutes.

2. The method according to claim 1, **characterised in that** in step c) the behaviour of each aquatic organism is recorded by simultaneously acquiring images by means of an imager.

3. The method according to claim 1 or 2, said benthic aquatic macroinvertebrates being selected from one or more species of different phylogenetic orders, preferably from one to three species.

4. The method according to any of the preceding claims, said benthic aquatic macroinvertebrates being selected from gastropods, leeches and/or crustaceans.

5. The method according to claim 4, said gastropods being of the genus Radix, preferably *Radix auricularia.*

6. The method according to claim 4 or 5, said leeches being of the Arhynchobdellida order, preferably of the Erpobdellidae family, for example *Erpobdella testacea.*

7. The method according to any of claims 4 to 6, said crustaceans being amphipods, preferably of the Gammaridae family, for example *Gammarus fossarum,*

8. The method according to any of claims 1 to 7, comprising a step prior to step a) of selecting benthic aquatic macroinvertebrates so that each species represents a homogeneous population in size, sex and non-parasiticity.

9. The method according to any of claims 1 to 8, each species of benthic aquatic macroinvertebrates coming from a single population.

10. The method according to any of claims 1 to 9, having a step, prior to step b), of stabling said benthic aquatic macroinvertebrates in order to acclimatise them to the measurement conditions.

11. The method according to any of claims 1 to 10, said benthic aquatic macroinvertebrates not being female benthic aquatic macroinvertebrates.

12. The method according to any of claims 1 to 11, said benthic aquatic macroinvertebrates having a survival rate of more than 80% following 30 days of diet.
